# NEUE EUROPÄISCHE PATENTSCHRIFT

(11) **EP 0 322 760 B2**
(45) Veröffentlichungstag und Bekanntmachung des Hinweises auf die Entscheidung über den Einspruch: **12.03.1997**
(45) Hinweis auf die Patenterteilung: 11.03.1992
(21) Anmeldenummer: 88121484.5
(22) Anmeldetag: 22.12.1988
(51) Int. Cl.: B01J 23/74, B01J 37/00, C07C 209/00

(54) **Verfahren zur Herstellung von Kobaltkatalysatoren**
Process for the preparation of cobalt catalysts
Procédé de préparation de catalyseurs à base de cobalt

(30) Priorität: 30.12.1987 DE 3744507
(43) Veröffentlichungstag der Anmeldung: 05.07.1989
(73) Patentinhaber: HOECHST AKTIENGESELLSCHAFT, 65926 Frankfurt am Main (DE)
(72) Erfinder: Horn, Gerhardt, Dr.Dipl.-Chem., D-4200 Oberhausen 11 (DE)

(56) Entgegenhaltungen:
- EP-A- 0 029 675
- EP-A- 0 151 986
- EP-A- 0 152 652
- EP-A- 0 168 096
- DE-A- 2 654 028
- DE-A- 2 702 327
- GB-A- 745 684
- US-A- 2 640 082
- US-A- 4 140 720
- US-A- 4 604 275

## Beschreibung

Die vorliegende Erfindung betrifft ein Verfahren zur Herstellung von Kobaltkatalysatoren, die bevorzugt als Hydrierkatalysatoren Anwendung finden. Sie haben sich insbesondere bei Reaktionen bewährt, an denen organische Stickstoffverbindungen als Ausgangsstoffe teilnehmen oder in denen sie als Produkt auftreten.

Kobalt und Nickel finden als Hydrierkatalysatoren umfangreiche Anwendung. Sie werden beispielsweise für die Hydrierung von Nitrilen und Dinitrilen zu den entsprechenden Aminen und Diaminen eingesetzt. Auch die reduktive Aminierung zur Herstellung primärer Amine, sie besteht darin, daß man ein Gemisch aus einem Aldehyd oder Keton und Ammoniak mit Wasserstoff behandelt, erfolgt in Gegenwart von Kobalt oder Nickel als Katalysatoren.

Mit Erfolg werden für die genannten Umsetzungen Raney-Kobalt und Raney-Nickel eingesetzt.

So laßt sich Diethylaminoacetonitril nach Winans und Adkins (Am.Soc. 55, 4167 (1933)) an Raney-Nickel mit 37 %iger Ausbeute zum Diamin hydrieren. Abgesehen davon, daß eine solche Ausbeute für ein technisches Verfahren zu gering ist, konnten die Angaben von Winans und Adkins nicht ohne weiteres reproduziert werden (vgl. Houben-Weyl 11/1, Seite 563).

In der GB-PS 745 684 ist ein Verfahren zur Herstellung von N,N-Dialkylaminoethylamin durch katalytisches Hydrieren von N,N-Dialkylaminoacetonitril bei überatmosphärischem Druck und bei Temperaturen unterhalb 110°C in Gegenwart von Raney-Kobalt beschrieben. Dieser Prozeß erfordert den Einsatzvon flüssigem Ammoniak und führt zu einer Ausbeute von 92 %.

Eine ausführliche Darstellung der reduktiven Aminierung von Carbonylverbindungen u.a. mit Raney-Nickel-Katalysatoren findet sich in Houben-Weyl, Methoden der organischen Chemie, Stuttgart 1957, Band XI/1, Seiten 602 ff.

Trotz ihrer hohen Aktivität haben Raney-Katalysatoren nur teilweise Eingang in die industrielle Praxis gefunden. Ein Grund hierfür ist ihre schwierige Handhabung. Sie können nur in suspendierter Form, also nicht als Festbett. eingesetzt werden. Zudem ist ihre Herstellung mit nicht unerheblichem Aufwand verbunden.

Es bestand daher die Aufgabe, Katalysatoren zu entwickeln, die die geschilderten Nachteile nicht aufweisen. Sie sollen einfach herzustellen sein, als Festbett verwendet werden können und eine den Raney-Kobalt-Katalysatoren vergleichbare Aktivität besitzen.

Diese Aufgabe wird durch die Erfindung gelöst.

Sie besteht in einem Verfahren zur Herstellung von Kobaltkatalysatoren durch Ausfällen von Kobaltcarbonat aus der wäßrigen Lösung eines Kobaltsalzes mit einer wäßrigen Alkalicarbonatlösung bei 20 bis 95°C, Filtrieren, Auswaschen und gegebenenfalls Formen der Katalysatormasse und anschließende Reduktion mit Wasserstoff. Es ist dadurch gekennzeichnet, daß die Reduktion zwischen 220 und 280°C in drei Stufen, bei von Stufe zu Stufe ansteigender Temperatur erfolgt, wobei in der ersten Stufe über einen Zeitraum von 1 bis 4 Stunden eine Temperatur von 220 bis 250°C, in der zweiten Stufe über einen Zeitraum von 1 bis 5 Stunden eine Temperatur von 245 bis 260°C und in der dritten Stufe über einen Zeitraum von 1 bis 5 Stunden eine Temperatur von 255 bis 280°C aufrechterhalten wird.

Ein ähnlicher verfahren ist in DE-A-2 654 028 offenbart, wobei die Reduktion anfangs bei der Temperatur von 320°C vorgenommen dann schließlich bei 360°C beendet wird.

Die Katalysatoren sind ausgezeichnet. zur Hydrierung organischer Verbindungen geeignet, insbesondere zur Hydrierung von Nitrilen zu Aminen und zur reduktiven Aminierung von Carbonylverbindungen.

Die Katalysatoren bestehen im reduzierten Zustand aus Kobalt. Nach einer bevorzugten Ausführungsform enthalten sie außerdem 0,25 bis 15 Gew.-% SiO₂, MnO₂, ZrO₂, Al₂O₃ oder MgO in Form der Oxide, Hydroxide oder Oxidhydrate. Diese Zusatzstoffe werden einzeln verwendet oderauch als Kombination von zwei oder mehreren Substanzen. Bevorzugt beträgt ihr Anteil 1 bis 8 und insbesondere 2 bis 5 Gew.-%. Alle vorstehenden Angaben in Gewichtsprozent beziehen sich auf die gesamte Katalysatormasse im wasserfreien Zustand vor der Reduktion.

Die oben aufgeführten Formeln dienen lediglich der Beschreibung der quantitativen Zusammensetzung der Katalyzatomasse, sie geben aber nicht unbedingt die genaue chemische Struktur der Zusatzstoffe wieder. Außer als Oxide können sie im nichtreduzierten wie auch im reduzierten Katalyator als Hydroxide und insbesondere als Oxidhydrate enthalten sein.

Die Wirkungsweise der Zusatzstoffe ist nicht in allen Einzelheiten geklärt. Versuchsergebnisse sprechen dafür, daß sie die Struktur des Katalysators, insbesondere seine Oberflächenstruktur gegenüber Sinterungseffekten bei hohen Temperaturen stabilisieren. Darüber hinaus erhöhen sie auch die mechanische Stabilität der aus der Katalysatormasse hergestellten Formkörper.

Zur Herstellung der Kobaltkatalysatoren fällt man bei 20 bis 95°C Kobaltcarbonat aus der wäßrigen Lösung eines Kobaltsalzes mit einer wäßrigen Alkalicarbonatlösung. Unter dem Begriff Kobaltcarbonat wird nicht nur die Verbindung CoCO₃ verstanden. Er schließt auch basische Carbonate und andere Produkte der Umsetzung wäßriger Lösungen von Kobaltsalz und Alkalicarbonat unter den gewählten Reaktionsbedingungen ein. Geeignete Kobaltsalze sind z.B. Kobaltnitrat, -chlorid, -sulfat, -acetat. Als Alkalicarbonate kommen insbesondere die Natrium oder die Kaliumverbindung in Betracht. Die Lösungen der Ausgangsstoffe enthalten Kobaltsalz bzw. Alkalicarbonat jeweils in Konzentrationen von 25 bis 150 g Co bzw. Alkalicarbonat/1 Lösung, Kobaltsalz und Alkalicarbonat können in äquimolaren Mengen miteinander umgesetzt werden, zweckmäßiger ist es jedoch, mit einem Alkalicarbonatüberschuß zu arbeiten. Bewährt hat es sich, 1,1 bis 1,5 und insbesondere 1,2 bis 1,3 Mol Alkalicarbonat je Mol Kobaltsalz zu verwenden.

Zur Herstellung einen oder mehrene Zusatzstoffe enthaltender Kobaltlkatalysatoren kann man die entsprechenden Substanzen in der Lösung des Kobaltsalzes oder in der Alkalicarbonatlösung suspendieren. Mit gleich gutem Erfolg kann man aber auch ein lösliches Salz des Zusatzstoffes vor der Fällung zur Kobaltsalzlösung geben und Kobaltcarbonat und Zusatzstoff gemeinsam ausfällen. Schließlich ist es auch möglich, die Kobaltcarbonatfällung getrennt vorzunehmen und anschließend den Zusatzstoff auf das Kobaltcarbonat aufzufällen.

So erhält man die als Zusatzstoffe verwendeten Verbindungen des Siliziums, z.B. durch Ansäuern einer Natriumsililtatlösung oder durch Hydrolyse eines Siliziumhalogenids. Zur Herstellung der Sauerstoffverbindungen des Mangans geht man von Mangan(II)-verbindungen wie dem Nitrat oder dem Chlorid aus und setzt sie mit Alkalihydroxid- oder Alkalicarbonat-Lösung um. In entsprechender Weise gelangt man zu den als Zusatzstoffe geeigneten Sauerstoffverbindungen des Zirkoniums z.B. durch Umsetzung von Zr(NO₃)₄ . 5H₂O oder ZrOCl₂ . 8H₂O mit Ammoniak, Alkalihydroxid oder -carbonat. Die Aluminium- und Magnesiumverbindungen erhält man ebenfalls durch Umsetzung ihrer in Wasser gelösten Verbindungen wie der Nitrate mit Ammoniak oder Alkalicarbonat (im Falle des Aluminiums) bzw. Alkalihydroxid oder -carbonat (im Falle des Magnesiums). Die Ausfällung der Sauerstoffverbindungen erfolgt bei 70 bis 95°C, insbesondere bei 80 bis 90°C.

Von großer Bedeutung für die Leistungsfähigkeit des Katalysators ist die Durchführung seiner Reduktion und damit seiner Aktivierung. Sie erfolgt in einem Temperaturbereich, der bei 220°C beginnt und 280°C, nicht übersteigt.

Als Reduktionsmittel verwendet man Wasserstoff, der mit einer Raumgeschwindigkeit von 200 bis 2000 1H₂ je 1 Katalysator und Stunde (200 bis 2000 V_{H} /V_{Kat} . h), bevorzugt 300 bis 1000 V_{H}/V_{Kat} . h und insbesondere 400 bis 700 V_{H}/V_{Kat} . h über die Katalysatorschüttung geleitet wird.

Es hat sich bewährt, in der ersten Stufe eine Temperatur von 220 bis 250°C, vorzugsweise 230 bis 240°C einzustellen und 1 bis 4, insbesondere 2 bis 3 Stunden aufrechtzuerhalten. In der zweiten Stufe erfolgt die Reduktion während 1 bis 5, insbesondere 2 bis 3 Stunden bei 245 bis 260°C, vorzugsweise 250 bis 255°C. Anschließend wird die Reduktion weitere 1 bis 5, insbesondere 2 bis 3 Stunden bei 255 bis 280°C, vorzugsweise bei 260 bis 270°C zu Ende geführt.

Innerhalb dieses vorgegebenen Rahmens kann man die Temperaturstufen vermehren und die Reduktionszeit innerhalb jeder Stufe entsprechend verringern. So ist es z.B. möglich, die erste Stufe in 2 Schritten aufzu leiten, wobei zunächst für die Dauer einer Stunde eine Temperatur von 220 bis 230°C und darauf für die Dauer von 1,5 Stunden eine Temperatur von 230 bis 240°C aufrechtemalten wird.

Der reduzierte Katalysator ist pyrophor und an der Luft selbstentzündlich. Zur besseren Handhabung läßt man daher einen N₂-Strom auf ihn einwirken, der Sauerstoff in einer Konzentration von etwa 0,5 bis etwa 1 Vol.-%, enthält. Durch diese Behandlung wird der Katalysator oberflächlich oxidiert; er ist in diesem Zustand an der Luft bis etwas 80°C stabil und nicht selbstentzündlich.

Die nach dem erfindungsgemäßen Verfahren hergestellten Katalysatoren haben eine nach BET gemessene Oberfläche von 10 bis 80, vorzugsweise 40 bis 60 m²/g. Ihr Porenvolumen beträgt 0,1 bis 0,6, vorzugsweise 0,3 bis 0,4 ml/g. Sie lassen sich in bekannter Weise zu Strängen und insbesondere zu Tabletten formen, die auch unter hoher Beanspruchung nur geringen Abrieb aufweisen.

Die Katalysatoren können in einem Lösungsmittel suspendiert eingesetzt werden. Besonders vorteilhaft ist ihre Verwendung als Festbett.

Die folgenden Beispiele beschreiben Herstellung und Verwendung der nach dem neuen Verfahren hergestellten Katalysatoren. Die Erfindung ist selbstverständlich nicht auf diese speziellen Ausführungsformen beschränkt.

### Beispiel 1

### Herstellung eines Kobaltkatalysators ohne Zusatzstoffe

In einer auf 90°C erhitzten Lösung von 800 g Na₂CO₃ in 7,5 l entionisiertem Wasser läßt man innerhalb von 2 min unter kräftigem Rühren eine auf 95°C erhitzte Lösung von 1852 g Co(NO₃)₂ . 6H₂O (≙ 375 g Co) in 7,5 l endonisiertem Wasser einfließen. Die Fällungssuspension weist einen pH-Wert von 8,2 bis 8,4 auf. Das Fällungsprodukt wird abfiltriert, mit etwa 90 l Kondensat-Wasser (Temperatur: 70°C) intensiv gewaschen bis die Leitfähigkeit des Waschwassers ≦ 100 µS ist. Das noch feuchte Katalysatorvorprodukt wird erneut suspendiert und darauf sprühgetrocknet oder extrudiert und 12 h im Luftstrom bei von 50 auf 75°C ansteigender Temperatur getrocknet. Das Extrudat weist folgende Kennwerte auf:

| | |
|---|---|
| Schüttgewicht | 600 bis 770 g/l |
| Co-Gehalt | etwa 53,5 Gew.-% |
| CO₃-Gehalt | 23,5 Gew.-% |
| Restalkali-Gehalt | etwa 0,06 bis 0,2 Gew.-% Na₂O |
| Restfeuchte | ≤ 10 Gew.-% |

Zur Reduktion werden über 0,5 l der extrudierten und getrockneten Katalysatormasse in einem Rohrreaktor (Durchmesser: 50 mm) bei 240°C während 2 h 200 l H₂/h geleitet. Darauf erhöht man die Temperatur auf 250°C und reduziert weitere 2 h mit 200 l H₂/h und führt die Reduktion durch Behandlung des Katalysators während weiterer 2 h mit 200 l H₂/h bei 260°C zu Ende.

Zur besseren Handhabung wird der reduzierte Katalysator zunächst 4 h bei Umgebungstemperatur und anschließend 2 h bei 70°C mit einem 0,7 Vol.-% O₂ enthaltenden N₂-Strom 1000 l N₂/l Katalysator . h behandelt. Der danach erhaltene Katalysator kann zu Pulver vermahlen oder zu Tabletten verpreßt werden.

### Beispiel 2:

### Herstellung eines Kobaltkatalysators mit Zusatzstoff.

In eine auf 90°C erhitzte Lösung von 840 g Na₂CO₃ in 7,5 l entionisiertem Wasser läßt man innerhalb von 2 min unter kräftigem Rühren eine auf 95°C erhitzte Lösung von 1852 g Co(NO₃)₂.6H₂O (≙ 375 g Co) und 85,73 g Mn (NO₃)₂.4H₂O in 7,5 l entionisiertem Wasser gleichmäßig einfließen. Es entsteht eine Suspension von Kobalt- und Mangancarbonat in Wasser mit einem pH-Wert von 8,2 bis 8,4. Das Fällungsprodukt wird abfiltriert und mit etwa 90 l 70°C heißem Kondensat-Wasser intensiv gewaschen, so daß die Leitfähigkeit des Waschwassers nach Beendigung des Waschvorganges kleiner als 100 µS ist.

Das noch feuchte Katalysatorvorprodukt wird erneut in entionisiertem Wasser suspendiert und darauf sprühgetrocknet oder extrudiert und 12 h im Luftstrom bei von 50 auf 75°C ansteigender Temperatur getrocknet. Das Extrudat weist folgende Kennwerte auf:

| | |
|---|---|
| Schüttgewicht | 600 bis 730 g/l |
| CO-Gehalt | etwa 52 Gew.-% |
| MnO₂-Gehalt | 4,1 Gew.-% |
| CO₃-Gehalt | etwa 22,5 Gew.-% |
| Restalkali-Gehalt | etwa 0,08 bis 0,3 Gew.-% Na2O |
| Restfeuchte | ≤ 10 Gew.-% |

Die Reduktion des Katalysators erfolgt wie in Beispiel 1 beschrieben.

### Beispiel 3: Hydrierung von Diethylaminoacetonitril

In einem beheizbaren Doppelmantelrohr von 28 mm Innendurchmesser und 3m Länge werden 1,8 l des in Beispiel 1 beschriebenen Kobalt-Katalysators in Form von Tabletten mit 6 mm Durchmesser als Festbett angeordnet. Man erhitzt auf 70°C und führt Wasserstoff unter einem Druck von 8 MPa sowie über eine Kolbenpumpe je Stunde kontinuierlich 600 ml einer Lösung von Diethylaminoacetonitril in Cyclohexan (15 Gew.-% Nitril, bezogen auf die Lösung) am Boden des Reaktionsrohres zu. Das am Reaktorkopf austretende Produkt enthält kein Diethylaminoacetonitril. Gaschromatographisch werden neben 86,5 % Losungsmittel 11 % Diethylaminoethylamin nachgewiesen.

### Beispiel 4:

### Hydrierung von Diethylaminoacetonitril

Im Reaktor des Beispiels 3 wird unter Verwendung von 1,8 l des Katalysators aus Beispiel 2 bei 60°C und einem H₂-Druck von 8 MPa H₂ Diethylaminoacetonitril in Form einer 30 Gew.-%igen Lösung (bezogen auf die Lösung) in Cyclohexan umgesetzt. Gleichzeitig leitet man dem Reaktor 2,5 Mol NH₃/Mol Nitril zu und erhöht. den Durchsatz auf 900 ml/h. Das Diethylaminoacetonitril ist vollständig umgesetzt. Das Reaktionsprodukt enthält nach gaschromatographischer Auswertung 73,2 % Cyclohexan und 25,1 % des Diethylaminoethylamins.

### Beispiel 5:

### Hydrierung von Diethylaminoacetonitril

Im Reaktor des Beispiels 3 wird bei 50°C und einem H₂-Druck von 8 MPa unverdünntes Diethylaminoacetonitrl umgesetzt. Je Mol Nitril führt man dem Reaktor 2,5 Mol NH₃ zu, der Durchsatz wird auf 180 ml/h ≙ V/Vh = 0,1 eingestellt. Das Nitril wird vollständig umgesetzt, das Reaktionsprodukt enthält nach gaschmmatographischer Analyse 89.7 % Diethylaminoethylamin, der Rest sind Spaltprodukte. Die Aufarbeitung des Reaktionsproduktes erfolgt in einer Kolonne mit 24 theoretischen Böden. Diethylaminoethylamin wird in mehr als 99 %iger Reinheit gewonnen.

## Patentansprüche

1. Verfahren zur Herstellung von Kobaltkatalysatoren durch Ausfällen von Kobaltcarbonat aus der wäßrigen Lösung eines Kobaltsalzes mit einer wäßrigen Alkalicarbonatlösung bei 20 bis 95°C, Filtrieren, Auswaschen und gegebenenfalls Formen der Katalysatormasse und anschließende Reduktion mit Wasserstoff, dadurch gekennzeichnet, daß die Reduktion zwischen 220 und 280°C in drei Stufen, bei von Stufe zu Stufe ansteigender Temperatur erfolgt, wobei in der ersten Stufe über einen Zeitraum von 1 bis 4 Stunden eine Temperatur von 220 bis 250°C, in der zweiten Stufe über einen Zeitraum von 1 bis 5 Stunden eine Temperatur von 245 bis 260°C und in der dritten Stufe über einen Zeitraum von 1 bis 5 Stunden eine Temperatur von 255 bis 280°C aufrechterhalten wird.

2. Verfahren nach Anspruch 1, dadurch gekennzeichnet, daß der Katalysator zusätzlich 0,25 bis 15 Gew.-%, bezogen auf die gesamte Katalyatormasse, SiO₂, MnO₂, ZrO₂, Al₂O₃ oder MgO einzeln oder als Kombination von zwei oder mehreren dieser Substanzen in Form der Oxide, Hydroxide oder Oxidhydrate enthält.

3. Verfahren nach Anspruch 2, dadurch gekennzeichent, daß SiO₂, MnO₂, ZrO₂, Al₂O₃ oder MgO in einer Menge von 1 bis 8 und insbesondere 2 bis 5 Gew.-%, bezogen auf die gesamte Katalysatormasse, im Katalysator enthalten sind.

4. Verfahren nach einem oder mehreren der Ansprüche 1 bis 3, dadurch gekennzeichnet, daß der Katalysator mit Wasserstoff bei einer Raumgeschwindigkeit von 200 bis 2000, vorzugsweise 300 bis 1000 und insbesondere 400 bis 700 l H₂ je l Katalysator und Stunde reduziert wird.

5. Verfahren nach einem oder mehreren der Ansprüche 1 bis 4, dadurch gekennzeichnet, daß die Reduktion in der ersten Stufe bei einer Temperatur von 230 bis 240°C, in der zweiten Stufe bei 250 bis 255°C und in der dritten Stufe bei 260 bis 270°C durchgeführt wird.

6. Verfahren nach Anspruch 5, dadurch gekennzeichnet, daß die Temperatur in der ersten Stufe, zweiten und dritten Stufe jeweils 2 bis 3 Stunden aufrechterhalten wird.

## Claims

1. A process for preparing cobalt catalysts by precipitating cobalt carbonate out of the aqueous solution of a cobalt salt with an aqueous alkali metal carbonate solution at 20 to 95°C, filtering, washing out and, optionally, shaping the catalyst mass and subsequently reducing with hydrogen, characterised in that reduction is performed at 220 to 280°C in three stages with the temperature increasing from stage to stage, a temperature of 220 to 250°C being maintained for a period of 1 to 4 hours in the first stage, a temperature of 245 to 260°C being maintained for a period of 1 to 5 hours in the second stage and a temperature of 255 to 280°C being maintained for a period of 1 to 5 hours in the third stage.

2. A process according to claim 1, characterised in that the catalyst contains in addition 0.25 to 15 wt % of SiO₂, MnO₂, ZrO₂, Al₂O₃ or MgO, related to the total catalyst mass, singly or as a combination of two or more of these substances, in the form of their oxides, hydroxides or hydrated oxides.

3. A process according to claim 2, characterised in that SiO₂, MnO₂, ZrO₂, Al₂O₃ or MgO are contained in the catalyst in an amount of 1 to 8 and in particular 2 to 5 wt %, related to the total catalyst mass.

4. A process according to one ore more of the claims 1 to 3, characterised in that the catalyst is reduced with hydrogen at a space velocity of 200 to 2000, preferably 300 to 1000 and in particular 400 to 700 l H₂ per litre of catalyst per hour.

5. A process according to one or more of the claims 1 to 4, characterised in that the reduction in the first stage is performed at a temperature of 230 to 240°C, in the second stage at 250 to 255°C, and in the third stage at 260 to 270°C.

6. A process according to claim 5, characterised in that the temperature in the first stage, second and third stage is maintained for 2 to 3 hours each.

## Revendications

1. Procédé de fabrication de catalyseurs au cobalt par précipitation de carbonate de cobalt à partir de la solution aqueuse d'un sel de cobalt par une solution aqueuse de carbonate alcalin à 20-95°C, filtration, lavage et éventuellement moulage de la masse de catalyseur et ensuite réduction par l'hydrogène, caractérisé en ce que la réduction est effectuée entre 220 et 280°C en trois étapes, à une température augmentant d'une étape à l'autre, en maintenant dans la première étape une température de 220 à 250°C sur une durée de 1 à 4 h, dans le deuxième étape une température de 245 à 260°C sur une durée de 1 à 5 h et dans la troisième étape une température de 255 à 280°C sur une durée de 1 à 5 h.

2. Procédé selon la revendication 1, caractérisé en ce que le catalyseur contient en outre 0,25 à 15% en poids, par rapport à la masse totale de catalyseur, de SiO₂, MnO₂, ZrO₂, Al₂O₃ ou MgO isolément ou en combinaison de deux ou plusieurs de ces substances sous forme des oxydes, hydroxydes ou oxydes hydratés.

3. Procédé selon la revendication 2, caractérisé en ce que SiO₂, MnO₂, ZrO₂, Al₂O₃ ou MgO sont présents dans le catalyseur en quantité de 1 à 8 et en particulier de 2 à 5% en poids, par rapport à la masse totale de catalyseur.

4. Procédé selon une ou plusieurs des revendications 1 à 3, caractérisé en ce que le catalyseur est réduit par l'hydrogéne à une vitesse spatiale de 200 à 2000, de préférence 300 à 1000 et en particulier 400 à 700 l de H₂ par litre de catalyseur et par heure.

5. Procédé selon une ou plusieurs des revendications 1 à 4, caractérisé en ce que la réduction est effectuée dans la première étape à une température de 230 à 240°C, dans la seconde étape à 250 à 255°C et dans la troisième étape à 260 à 270°C.

6. Procédé selon la revendication 5, caractérisé en ce que la température est maintenue dans la première étape, la seconde étape et la troisième étape chaque fois pendant 2 à 3 h.
